Europäisches Patentamt

European Patent Office

Office européen des brevets

(11) Publication number: **0 207 676 B1**

# EUROPEAN PATENT SPECIFICATION

(45) Date of publication of patent specification: **01.06.94**

(51) Int. Cl.5: **C08B 30/18**, C13K 1/06, A61M 1/28, A61K 31/715

(21) Application number: **86304624.9**

(22) Date of filing: **16.06.86**

The file contains technical information submitted after the application was filed and not included in this specification

(54) **Polymers for use in continuous peritoneal dialysis.**

(30) Priority: **22.06.85 GB 8515842**
**02.05.86 GB 8610841**

(43) Date of publication of application:
**07.01.87 Bulletin 87/02**

(45) Publication of the grant of the patent:
**01.06.94 Bulletin 94/22**

(84) Designated Contracting States:
**AT BE CH DE FR GB IT LI LU NL SE**

(56) References cited:
**EP-A- 0 153 164**
**WO-A-82/03329**

**CHEMICAL ABSTRACTS, vol. 85, 1976, page 410, abstract no. 141580f, Columbus,Ohio, US; & JP-A-76 88 645 (AJINOMOTO CO., INC.) 03-08-1976**

**CHEMICAL ABSTRACTS, vol. 99, 1983, page 619, abstract no. 105608b, Columbus,Ohio, US; S. KIKUMOTO et al.: "Naegeli amylodextrin. Large scale preparation of fractions by step-wise precipitation using organic solvents", & DENPUN KAGAKU, 30(1), 69-75**

fractions by step-wise precipitation using organic solvents", & DENPUN KAGAKU1983, 30(1), 69-75

(73) Proprietor: **M L LABORATORIES PLC**
**Marcol House, 293 Regent Street**
**London W1R 7PD(GB)**

(72) Inventor: **Alsop, Ranulph Michael**
**28 Windemere Drive**
**Alderley Edge Cheshire(GB)**
Inventor: **Forrester, Raymond Brian**
**7 Alderley Close**
**Sandbach Cheshire(GB)**
Inventor: **Manning, David John**
**Hillview**
**Under Rainow Road**
**Timbersbrook**
**Congleton Cheshire(GB)**

(74) Representative: **Harrison, Michael Robert et al**
**DIBB LUPTON BROOMHEAD and PRIOR**
**117 The Headrow**
**Leeds LS1 5JX (GB)**

**Description**

This invention relates to a new form of polymer, a method for its production and compositions containing it.

Maltodextrins (glucose polymers) are produced by the hydrolysis of pure starch isolated from various natural products, e.g. wheat, rice and tapioca. In a typical process a pure isolated starch is produced by a multi-stage separation process involving removal of protein, oil, fibre and glutens before being hydrolysed.

As no single number can adequately characterise the molecular weight of a polymer, such as a maltodextrin, various averages are used. The most commonly used are the weight average molecular weight ($\overline{M}_w$) and the number average molecular weight ($\overline{M}_n$):

$$\overline{M}_w = \frac{\sum n_i M_i^2}{\sum n_i M_i}$$

$$\overline{M}_n = \frac{\sum n_i M_i}{\sum n_i}$$

where $n_i$ is the number of molecules of molecular weight $M_i$. $\overline{M}_w$ is particularly sensitive to changes in the high-molecular-weight content of the maltodextrin polymer whilst $\overline{M}_n$ is largely influenced by changes in the low molecular weight of the sample.

We have now found that it is possible to monitor starch hydrolysis and in particular to stop the hydrolytic action when the hydrolysate contains the maximum amount of molecules in the desired molecular weight range. The monitoring may be carried out by a technique known as size exclusion chromatography. Furthermore, fractionation of the starch hydrolysate can be monitored by size exclusion chromatography and a weight average molecular weight, a number average molecular weight and a molecular weight distribution of the products can be determined using chromatographic columns calibrated with dextran standards (Alsop et al Process Biochem 2 10-15 (1977) and Alsop et al J. Chromatography 246, 227-240, (1982)).

We have also found a method for optimising the yield of a glucose polymer with a preselected molecular weight range.

Glucose polymers are often characterised by the expression "degree of polymerisation" (DP). In this terminology a product may be described as having 20% of its weight comprising molecules with a DP greater than 10, ie. 20% has a molecular weight greater than 1656 (a polymer comprising 10 glucose units).

British Patent Application 2132914A describes a glucose polymer mixture having at least 15% by weight of glucose polymers of DP greater than 12 for use in continuous ambulatory peritoneal dialysis (CAPD). PCT/US Application 82/00774 describes a CAPD solution comprising glucose polymers of DP of at least 4.

European Patent Application 0076355 A2 discloses glucose polymer mixtures having at least 99% of glucose polymers of DP less than 12 for use in CAPD.

European patent specification No 153,164 (Milner) also discloses glucose polymer mixtures for use in peritoneal dialysis, the mixtures containing more than 50% by weight of glucose polymers of D.P. greater than 12 (in the claims) and as low as 15% of polymers of D.P. greater than 12 (in the examples). Milner makes no mention of the number average molecular weight values for these mixtures, and the mixtures which he discloses span an extremely wide range of possible $M_n$ values.

It has now been surprisingly found that a small sub-range of the wide range of polymer mixtures disclosed by Milner is particularly effective for use in peritoneal dialysis. This sub-range is defined by reference to the $M_n$ values for the mixtures, which values have been found to be critical in predicting the effectiveness of the mixtures in, in particular, CAPD.

According to the present invention there is provided a peritoneal dialysis composition containing an osmotic agent which is a mixture of glucose polymers, wherein at least 50% by weight of the mixture comprises polymers having molecular weights in the range of from 5,000 to 30,000, and wherein the mixture has a weight average molecular weight of from 5,000 to 50,000 and a number average molecular weight of from 2,900 to 8,000, both the weight average molecular weight and the number average molecular weight being determined using chromatographic columns calibrated with dextran standards.

The mixture of glucose polymers preferably contains less than 5% by weight of polymers of molecular weight greater than 100,000. More preferably, it contains no more than 20% by weight of polymers having molecular weights of from 800 to 10,000.

The composition may additionally comprise a pharmaceutically acceptable adjuvant, diluent or carrier.

It has surprisingly been found that certain polydisperse glucose polymer mixtures of high molecular weight are useful in medicine, e.g. in CAPD and in prevention of post-operative adhesions.

The glucose polymer mixture of the present invention has at least 50% by weight of polymers of molecular weight in the range 5000 to 30000.

We particularly prefer a glucose polymer mixture wherein at least 80% by weight of the polymers are of molecular weight in the range 5000 to 50,000.

We prefer the glucose polymer mixture to have a weight average molecular weight in the range of from 5000 to 100000, preferably of from 5000 to 50000, more preferably of from 12000 to 25000, and most preferably of from 14000 to 20000.

We prefer the content of mono-, di-, and tri-saccharide compounds present in the glucose polymer mixture to be less than 5% by weight, more preferably less than 2% and most preferably 0% by weight. By 0% we mean an amount which is undetectable by conventional methods.

We further prefer that the content of glucose polymers with molecular weight greater than 100000 in the glucose polymer mixture should be less than 5%, preferably less than 3% and most preferably less than 1% by weight.

We prefer the glucose polymers to be substantially free from endotoxins and nitrogenous contaminants arising from the original starch, or from the enzyme preparations used for its hydrolysis.

We particularly prefer the endotoxin level to be less than 0.25 endotoxin units/ml, more preferably less than 0.12 endotoxin units/ml and most preferably less than 0.06 endotoxin units/ml as determined by the Limilus Lysate Test (US Pharmacopoeia).

We prefer the nitrogen content of the glucose polymers to be less than 0.01% w/w, more preferably less than 0.001% w/w and most preferably zero as determined by the Kjeldahl method (British Pharmacopoeia).

We also prefer the glucose polymers to be substantially free of undesirable metals, e.g. aluminium. Thus we prefer the level of aluminium to be less than 500 ppb, more preferably less than 200 ppb and most preferably less than 100 ppb.

We also prefer an aqueous solution comprising 10% w/v of the glucose polymer to be substantially clear and colourless. Thus we prefer such a solution to have a turbidity value of less than 30 EEL units (US Pharmacopoeia), more preferably less than 20 EEL units and most preferably less than 10 EEL units. We also prefer such a solution to have no substantially visible colour. We particularly prefer the solution to have a visible colour of less than 10 APHA Hazen units and more preferably less than 5 APHA Hazen units. The content of colour precursors such as 5-hydroxymethyl furfural can be measured by absorption of ultraviolet light of wavelength 275 or 284nm. We prefer the absorbance to be less than 0.5, more preferably less than 0.25 and most preferably less than 0.15. The transmission of ultraviolet light measured at a wavelength of 430 nm is preferably greater than 90% and more preferably greater than 95%.

We also disclose a glucose polymer (I) having up to 20% by weight of glucose polymers with a molecular weight of from 800 to 10,000, preferably of from 1500 to 4000. We particularly prefer a glucose polymer (I) having up to 20% by weight of glucose polymers with a molecular weight of from 1500 to 2500, more preferably up to 10% by weight and most preferably up to 7% by weight.

We also disclose a method for the production of a glucose polymer (I), which comprises

a) fractional precipitation of an aqueous solution of a glucose polymer containing polymer (I) with a water miscible solvent, and/or

b) filtration of an aqueous solution of a glucose polymer containing polymer (I) through membranes possessing an appropriate molecular weight cut-off range. The molecular weight cut-off range may be determined empirically.

In process a) the process parameters used are interdependent and each parameter may vary depending upon the desired quality of the product, the desired molecular weight range, etc. The water miscible solvent may be an alcohol, eg an alkanol, such as ethanol. The solvent may be present in an aqueous solution which is mixed with an aqueous glucose polymer. The concentration of the solvent in the aqueous solution before mixing may be from 60 to 100% v/v, preferably from 75 to 90% v/v, and most preferably about 85% v/v.

The concentration of the aqueous glucose polymer solution before mixing may be from 0 to 80% w/v, preferably from 15 to 65% w/v, and most preferably from 30 to 40% w/v.

The fractionation may be carried out at a temperature of from 10 to 40°C and more preferably from 20 to 30°C.

In process b) the type of membrane material used may vary with the particular molecular weight distribution which is desired. A chemically inert plastics material may be used for the membrane, eg. a cellulose acetate or polytetrafluoro-ethylene. We particularly prefer to use a material which is mechanically stable at high temperatures and pressures, eg. a polysulphone.

A series of membranes may be used consecutively such that both a high and a low molecular weight fractionation is carried out. The membrane fractionation may be carried out at elevated temperature sufficient to prevent bacteriological contamination. We prefer the fractionation to be carried out at a temperature of from 0 to 90°C, preferably from 20 to 80°C, and most preferably from 65° to 75°C.

The feed solution may be of a concentration of from 1.0 to 30.0% w/v, preferably from 5 to 15% w/v and most preferably about 10% w/v.

The glucose polymer starting material is preferably prepared by a method, e.g. hydrolysis, designed to optimise the proportion of polymer (I), and the progress of that method is preferably monitored by size exclusion chromotography. Any starch may be used in the hydrolysis but we prefer to use a cornstarch.

The molecular weight distribution of the fractions may be determined using the chromatographic techniques described by Alsop et al J. Chromatography 246, 227-240 (1982). The optical rotation of the various solutions produced may also be used to identify the concentrations of the polymer contained by the solutions.

The high molecular weight waste products from the fractionations may be further hydrolysed to produce further quantities of lower molecular weight products which can be fractionated. The low molecular weight waste products may be useful in the production of glucose syrups.

Before, during and/or after the fractionation of process a) or b) the polymer may be purified. The purification may be to remove undesirable colour or to remove contaminants, for example proteins, bacteria, bacterial toxins, fibres or trace metals, eg aluminium. Any conventional purification technique may be applied, for example, filtration and/or absorption/adsorption techniques such as ion exchange or charcoal treatment.

The product of the fractionation of process a) or b) may be packaged and transported as a syrup or solution, for example an aqueous solution. However, we prefer the product to be in a solid form, preferably a powder, and most preferably spray dried granules.

The glucose polymer (I) is useful in a wide variety of medical indications, e.g. peritoneal dialysis, as a nutritional agent or for the prevention of post-operative adhesions etc.

According to the invention we also provide a pharmaceutical composition comprising a glucose polymer mixture, wherein at least 50% of the mixture comprises polymers of a molecular weight in the range 5000 to 30000 and wherein the mixture has a weight average molecular weight of from 5,000 to 50,000 and a number average molecular weight of from 2,890 to 8,000, in admixture with a pharmaceutically acceptable adjuvant, diluent or carrier.

Any composition for in CAPD preferably comprises physiologically acceptable electrolytes, eg. sodium, potassium, calcium and magnesium, in order to prevent the transfer of desirable electrolytes from the serum to the peritoneum. The amounts may vary depending upon the requirements of any individual patient and are generally sufficient to provide an osmolarity of from about 240 to 275 mOsm/litre (see Example A).

According to the invention we also provide a physiologically acceptable polysaccharide (II) with an osmolarity of less than 160 mOsm/litre, preferably less than 110 mOsm/litre more preferably less than 90 mOsm/litre and most preferably less than 20 mOsm/litre, which is capable of being used in solution in the dialysis of normal human serum. By normal human serum we mean serum with an osmolarity of between 280 and 290 mOsm/litre at 37°C. The polysaccharide (II) preferably has the molecular weight and other parameters described above with respect to glucose polymer (I). Any suitable polysaccharide may be used but we prefer the polysaccharide to be a glucose polymer (I).

The polysaccharide (II) may be prepared by any of the processes hereinbefore described or by conventional processes known per se.

We also provide a composition capable of dialysing normal human serum comprising a polysaccharide (II) and having an osmolarity somewhat greater than normal serum. The osmolarity of the composition is preferably less than 400 mOsm/litre, more preferably less than 350 mOsm/litre and most preferably less than 330 mOsm/litre at 37°C. We particularly prefer a composition with an osmolarity less than 300 mOsm/litre at 37°C.

The composition may be in solid form, eg suitable for extemporaneous production of a solution, or it may be a liquid, eg in the form of an aqueous solution. The composition preferably includes pharmacologically acceptable electrolytes. Such electrolytes may include appropriate ions , eg of sodium, potassium, calcium, magnesium and chloride; buffers, eg. lactate, acetate or bisulphite; or other additives, such as amino acids, polyols or insulin.

The polymer (I) and the polysaccharide (II) are advantageous over the prior art. The long term use of high osmolarity glucose solutions in peritoneal dialysis can result in irreversible changes to the peritoneal membrane due to the continuous high pressure differentials across the peritoneum. When a glucose solution with a low osmolarity is used in CAPD for greater than four hours glucose may be lost from the peritoneum to the serum, this is undesirable, particularly in diabetic patients. The present invention provides a method of applying an osmotic pressure over the peritoneum for greater than four hours without causing damage to the peritoneum whilst preventing appreciable loss of polysaccharide to the serum from the peritoneum and maintaining the flow of water from the serum to the peritoneum.

The invention will now be described by way of example only and by reference to the attached drawings in which

Figure 1 is a flow diagram of the process described in Example 1;

Figure 2 is a flow diagram of the process described in Example 2;

Figure 3 is a flow diagram of the process described in Example 3;

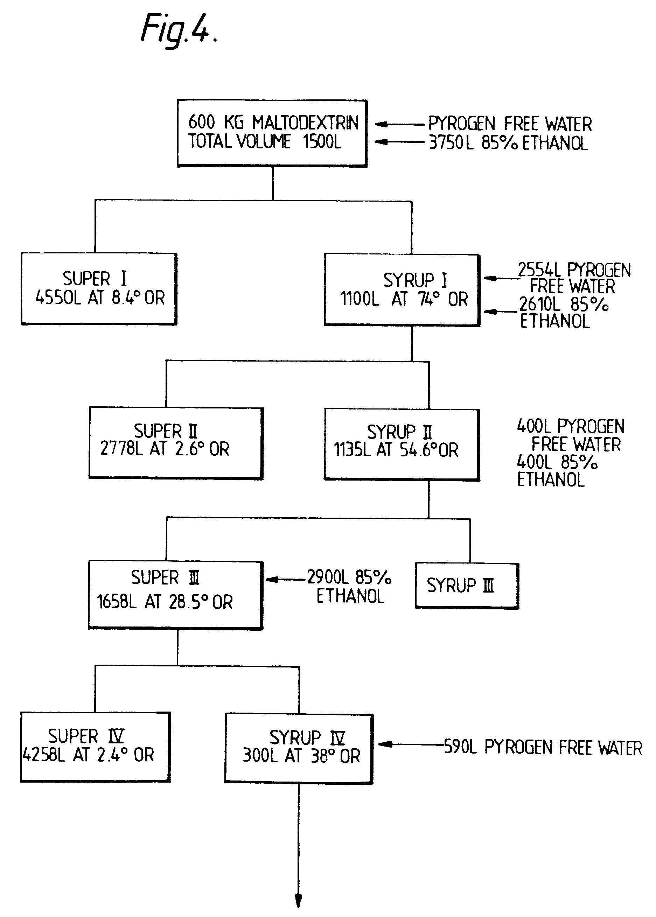

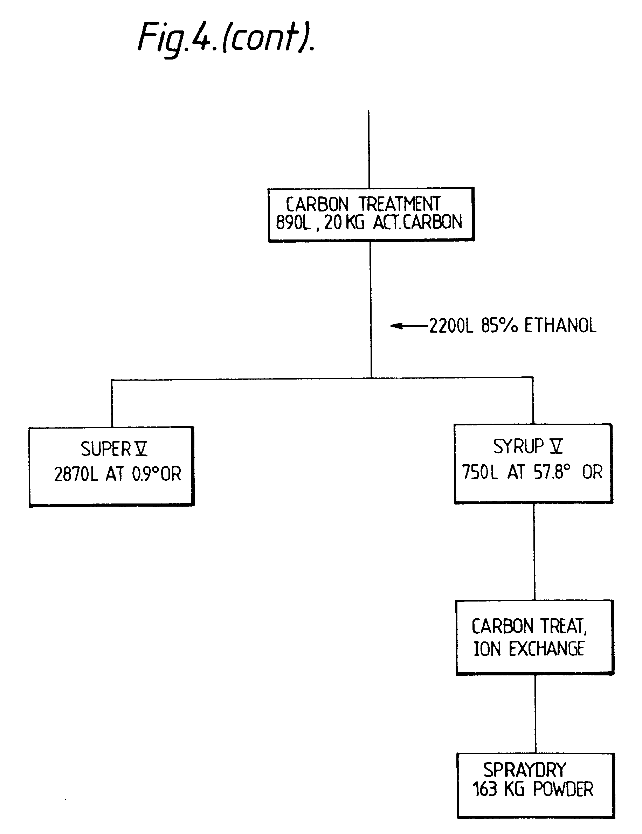

Figure 4 is a flow diagram of the process described in Example 4; and

Figure 5 is a flow diagram of the process described in Example 5.

In the Examples OR means optical rotations.

The molecular weight distribution of the starch hydrolysate starting material which was used in Examples 1 and 2 is shown in Table 1. The starting material was found to have an $\overline{M}_w$ of 6309 and an $\overline{M}_n$ of 401.

## Example 1

### Ethanol Fractionation

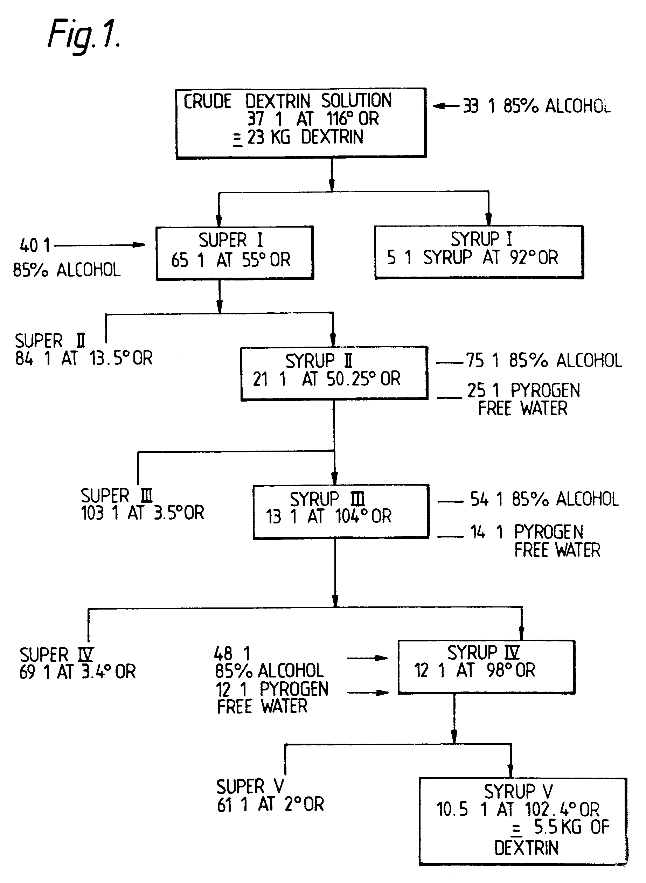

The fractionation procedure used to isolate the required molecular weight distribution of a maltodextrin syrup is given in Figure 1. The precise technique to be used will of course be varied to take account of the quality and molecular weight distribution of the maltodextrin used as the starting material.

Aqueous ethanol (33 l at 85%v/v) was added, with stirring, to 37 l of a maltodextrin syrup (at 116° OR = 23kg, dissolved maltodextrins). After settling the resulting Syrup I (5 l at 92° OR) was drawn from the bottom outlet of the fractionator.

Aqueous ethanol (40 l at 85% v/v) was added, with stirring, to the Supernatant I. After settling the Supernatant II (84 l at 13.5° OR) was decanted.

Aqueous ethanol (75 l at 85% v/v) and pyrogen free water (25 l) were added, with stirring, to the Syrup II (46 l at 50.25° OR). After settling the Supernatant III (103 l at 3.5° OR) was decanted.

Aqueous ethanol (54 l at 85% v/v) and pyrogen free water (14 l) were added, with stirring, to the resulting Syrup III (13 l at 104° OR). After settling the Supernatant IV (69 l at 3.4° OR) was decanted.

Aqueous ethanol (48 l at 85% v/v) and pyrogen free water (12 l) were added with stirring, to the resulting Syrup IV (12 l at 98° OR). After settling the required maltodextrin fraction, Syrup V, (10.51 at 102.4° OR ≡ 5.5kg dissolved maltodextrins) was drawn off. This represents 23.9% recovery of the maltodextrins present in the initial syrup. 3.8kg of Syrup V was dissolved in pyrogen free water (25 l) and refluxed with stirring in the presence of 0.4kg of activated carbon (Norit UK, GSX grade). The carbon was removed by filtration and the resulting syrup was used to prepare peritoneal dialysis solutions.

The $\overline{M}_w$ of the product maltodextrin after carbon treatment was 18949 and the $\overline{M}_n$ was 6316. The molecular weight distribution is shown in Table 2, 61% of the product lies within the range 5000 to 30000.

## Example 2

### Ethanol Fractionation

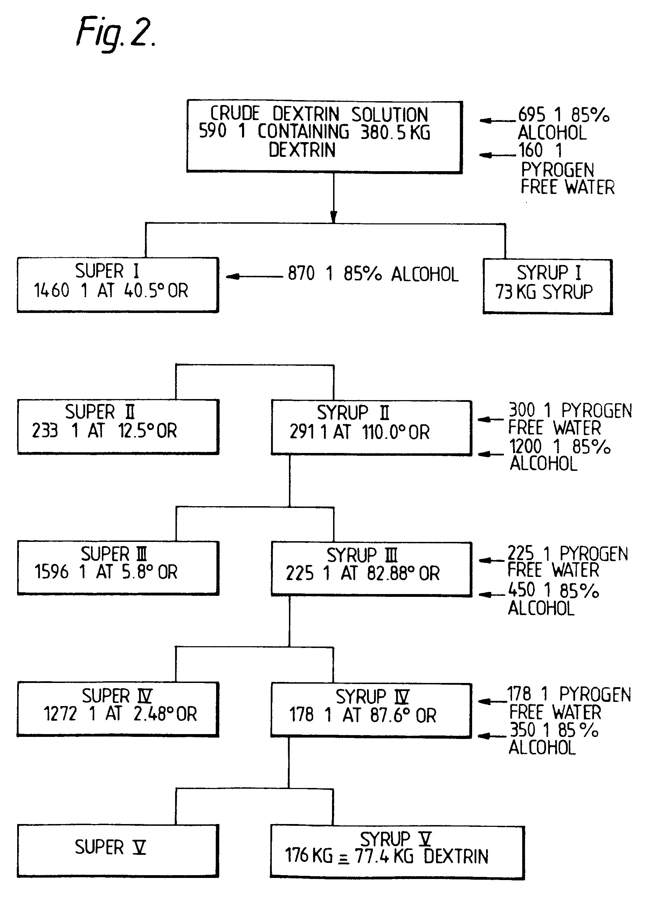

The procedure of Example 1 was repeated using the quantities shown in Figure 2. However, the carbon treatment was carried out by adding the activated carbon (Norit UK, grade GSX 5kg) to the alcoholic Syrup V. The alcohol was removed by steam distillation and the carbon by depth filtration (Carlson Ford grade NA90). The resulting syrup was then spray dried.

The $\overline{M}_w$ of the product maltodextrin was 12027 and the $\overline{M}_n$ was 3447. The molecular weight distribution is shown in Table 3, 60% of the product lies within the range 5000 to 30000.

The $\overline{M}_w$ of the product maltodextrin after carbon treatment was 12027 and the $\overline{M}_n$ was 3447. The molecular weight distribution is shown in Table 3, 60% of the product lies within the range 5000 to 30000.

### Example 3

#### Ethanol Fractionation

The molecular weight distribution of the starting material is shown in Table 4. The starting material had an $\overline{M}_w$ of 11534 and an $\overline{M}_n$ of 586.

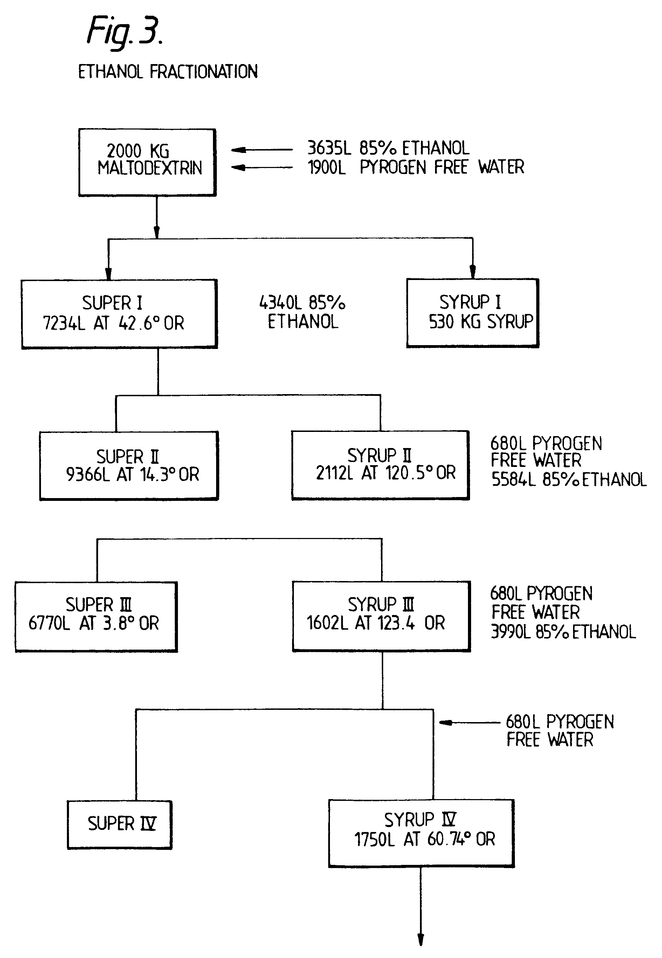

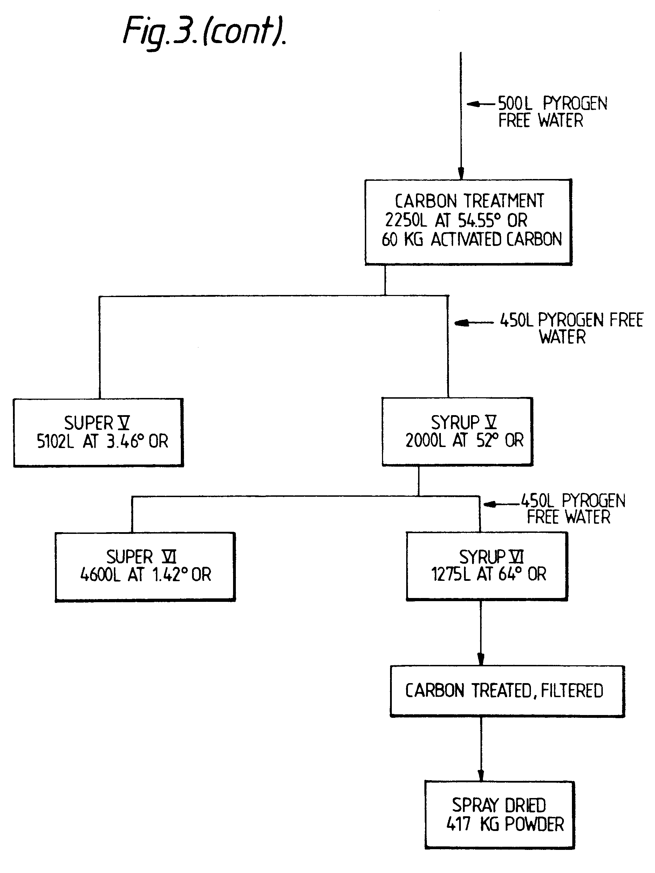

The procedure of Example 1 was repeated using the quantities shown in Figure 3. However, the carbon treatment was carried out by adding the activated carbon (Norit UK, Grade GSX 60kg) to the alcoholic syrup IV. The activated carbon was filtered off by depth filtration (Carlson Ford Grade 'O' pads). A further carbon treatment was carried out on the syrup VI (15kg Norit UK Grade GSX, filtered off using Carlson Ford Grade NA90 pads) during ethanol removal by steam distillation. The ethanol-free syrup was spray dried.

The $\overline{M}_w$ of the product maltodextrin was 21838 and the $\overline{M}_n$ was 7105. The molecular weight distribution is shown in Table 5, 58% of the product lies within the range 5000 to 30000.

### Example 4

#### Ethanol Fractionation

The molecular weight distribution of the starting material is shown in Table 6. The starting material had an $\overline{M}_w$ of 12636 and an $\overline{M}_n$ of 639.

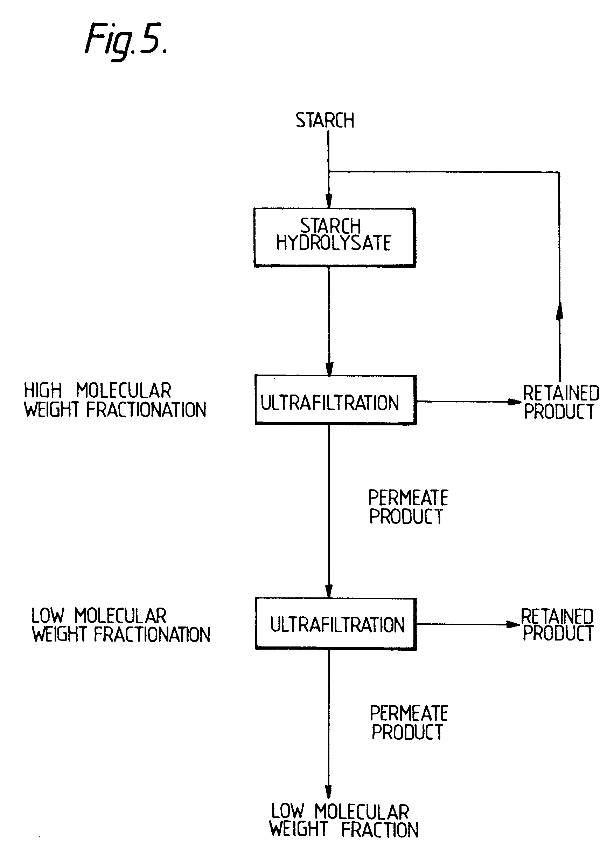

The procedure of Example 1 was repeated using the quantities shown in Figure 5. The carbon treatment was carried out by adding activated carbon (Norit UK, Grade GSX, 20kg) to the alcoholic syrup IV. The carbon was filtered by depth filtration (Carlson Ford Grade 'O' pads). Ethanol was removed from the final syrup (syrup V) by steam distillation and the aqueous product ion exchanged (mixed bed system), and spray dried. The mixed bed resin was Duolite A1725 in the hydroxyl form and C225H in the chloride form. (Duolite is a trade mark).

The $\overline{M}_w$ of the product maltodextrin was 22020 and The $M_n$ was 7767. The molecular weight distribution is shown in Table 7; 60% of the product lies within the range 5000 to 30000.

### Example 5

#### a) Membrane Fractionation

A high molecular weight fractionation was carried out by passing 2.0kg of starch hydrolysate, (molecular weight distribution, see Table 8), as a 10% w/v solution (20 litres) through a series of membranes. Polysulphone membranes with an approximate molecular weight cut-off of 25,000 and an area of $0.144m^2$ were used. The feed flowrate was 6.6 litres/min at a temperature of $70°C$. The total solids level of the retained liquid was maintained at 10% w/v and the low molecular weight species were washed through the membrane. After 5.5 hours the concentration of carbohydrate in the permeate product stream leaving the ultrafiltration module was undetectable (see Table 9) and the process was terminated. The high molecular weight residues were recovered from the membrane (0.384 kg, 19.2%) and the permeative low molecular weight product was isolated from the permeate (1.61 kg, 80.6%).

The molecular weight distribution of the permeate is shown in Table 10. $M_w$ was found to be 4906 and $M_n$ determined as 744.

#### b) Ethanol Fractionation

1.7kg of maltodextrin from Example 5a) in 53 litres of pyrogen free water was mixed with 132.5 litres of aqueous ethanol (85% v/v).

The syrup from the fractionation had an $M_w$ of 19712 and an $M_n$ of 4798. The molecular weight distribution is shown in Table 11; 55% of the product lies within the range 5000 to 30000.

6

EP 0 207 676 B1

Example A

Two examples of peritoneal dialysis solutions are shown below. The ionic electrolytes behave ideally and therefore 1 mOsm/l is equivalent to 1 mmol/l.

| | 1 | 2 |
|---|---|---|
| Sodium (mOsm/l) | 131 | 138 |
| Potassium (mOsm/l) | 0 | 0 |
| Calcium (mOsm/l) | 1.8 | 1.78 |
| Magnesium (mOsm/l) | 0.75 | 0.75 |
| Chloride (mOsm/l) | 91 | 90 |
| Lactate (mOsm/l) | 45 | 45 |
| Acetate (mOsm/l) | - | - |
| Bisulphite (mOsm/l) | - | - |
| Total Electrolyte Osmolarity (mOsm/l) | 269.6 | 275.5 |
| Glucose polymer (I) (mOsm/l) | 12.9 (50g/l) | 12.9 (50g/l) |
| Total Osmolarity | 282.5 | 288.4 |

Table 1

| Molecular Weight Distribution | |
|---|---|
| MOLECULAR WEIGHTS | INTEGRAL DISTRIBUTION |
| 165 | 0.00 |
| 167 | 2.50 |
| 172 | 5.00 |
| 178 | 7.50 |
| 184 | 10.00 |
| 191 | 12.50 |
| 199 | 15.00 |
| 207 | 17.50 |
| 216 | 20.00 |
| 226 | 22.50 |
| 237 | 25.00 |
| 249 | 27.50 |
| 262 | 30.00 |
| 276 | 32.50 |
| 291 | 35.00 |
| 307 | 37.50 |
| 326 | 40.00 |
| 346 | 42.50 |
| 366 | 45.00 |
| 391 | 47.50 |
| 419 | 50.00 |
| 446 | 52.50 |
| 488 | 55.00 |
| 532 | 57.50 |
| 598 | 60.00 |
| 681 | 62.50 |
| 837 | 65.00 |
| 1099 | 67.50 |
| 1570 | 70.00 |
| 2328 | 72.50 |
| 3436 | 75.00 |
| 4915 | 77.50 |
| 6789 | 80.00 |
| 7135 | 82.50 |
| 12074 | 85.00 |
| 13825 | 87.50 |
| 20735 | 90.00 |
| 27447 | 92.50 |
| 37044 | 95.00 |
| 53463 | 97.50 |
| 199559 | 100.00 |

EP 0 207 676 B1

Table 2

| Molecular Weight Distribution | |
|---|---|
| MOLECULAR WEIGHTS | INTEGRAL DISTRIBUTION |
| 296 | 0.00 |
| 1231 | 2.50 |
| 1756 | 5.00 |
| 2279 | 7.50 |
| 2795 | 10.00 |
| 3291 | 12.50 |
| 3771 | 15.00 |
| 4246 | 17.50 |
| 4722 | 20.00 |
| 5203 | 22.50 |
| 5696 | 25.00 |
| 6196 | 27.50 |
| 6718 | 30.00 |
| 7247 | 32.50 |
| 7809 | 35.00 |
| 8378 | 37.50 |
| 8986 | 40.00 |
| 9607 | 42.50 |
| 10272 | 45.00 |
| 10960 | 47.50 |
| 11695 | 50.00 |
| 12472 | 52.50 |
| 13295 | 55.00 |
| 14184 | 57.50 |
| 15126 | 60.00 |
| 16162 | 62.50 |
| 17274 | 65.00 |
| 18499 | 67.50 |
| 19872 | 70.00 |
| 21352 | 72.50 |
| 23122 | 75.00 |
| 25084 | 77.50 |
| 27319 | 80.00 |
| 30070 | 82.50 |
| 33400 | 85.00 |
| 37527 | 87.50 |
| 42867 | 90.00 |
| 50412 | 92.50 |
| 61686 | 95.00 |
| 82648 | 97.50 |
| 288182 | 100.00 |

9

Table 3

| Molecular Weight Distribution | |
|---|---|
| MOLECULAR WEIGHTS | INTEGRAL DISTRIBUTION |
| 183 | 0.00 |
| 484 | 2.50 |
| 874 | 5.00 |
| 1292 | 7.50 |
| 1695 | 10.00 |
| 2082 | 12.50 |
| 2460 | 15.00 |
| 2836 | 17.50 |
| 3215 | 20.00 |
| 3595 | 22.50 |
| 3986 | 25.00 |
| 4382 | 27.50 |
| 4786 | 30.00 |
| 5204 | 32.50 |
| 5627 | 35.00 |
| 6072 | 37.50 |
| 6519 | 40.00 |
| 6994 | 42.50 |
| 7473 | 45.00 |
| 7982 | 47.50 |
| 8499 | 50.00 |
| 9048 | 52.50 |
| 9611 | 55.00 |
| 10212 | 57.50 |
| 10836 | 60.00 |
| 11502 | 62.50 |
| 12208 | 65.00 |
| 12955 | 67.50 |
| 13777 | 70.00 |
| 14637 | 72.50 |
| 15626 | 75.00 |
| 16708 | 77.50 |
| 17905 | 80.00 |
| 19298 | 82.50 |
| 20957 | 85.00 |
| 22960 | 87.50 |
| 25476 | 90.00 |
| 29002 | 92.50 |
| 34287 | 95.00 |
| 44550 | 97.50 |
| 299523 | 100.00 |

Table 4

| Molecular Weight Distribution | |
| --- | --- |
| MOLECULAR WEIGHTS | INTEGRAL DISTRIBUTION |
| 146 | 0.00 |
| 157 | 2.50 |
| 173 | 5.00 |
| 192 | 7.50 |
| 213 | 10.00 |
| 235 | 12.50 |
| 259 | 15.00 |
| 285 | 17.50 |
| 313 | 20.00 |
| 343 | 22.50 |
| 378 | 25.00 |
| 411 | 27.50 |
| 450 | 30.00 |
| 489 | 32.50 |
| 536 | 35.00 |
| 583 | 37.50 |
| 636 | 40.00 |
| 695 | 42.50 |
| 755 | 45.00 |
| 837 | 47.50 |
| 920 | 50.00 |
| 1036 | 52.50 |
| 1161 | 55.00 |
| 1350 | 57.50 |
| 1590 | 60.00 |
| 1919 | 62.50 |
| 2393 | 65.00 |
| 3094 | 67.50 |
| 4176 | 70.00 |
| 5731 | 75.00 |
| 7802 | 75.00 |
| 10354 | 77.50 |
| 13393 | 80.00 |
| 17014 | 82.50 |
| 21436 | 85.00 |
| 27030 | 87.50 |
| 34348 | 90.00 |
| 44586 | 92.50 |
| 60087 | 95.00 |
| 89965 | 97.50 |
| 578156 | 100.00 |

Table 5

| Molecular Weight Distribution | |
|---|---|
| MOLECULAR WEIGHTS | INTEGRAL DISTRIBUTION |
| 1394 | 2.50 |
| 2060 | 5.00 |
| 2644 | 7.50 |
| 3199 | 10.00 |
| 3751 | 12.50 |
| 4299 | 15.00 |
| 4856 | 17.50 |
| 5421 | 20.00 |
| 6003 | 22.50 |
| 6597 | 25.00 |
| 7208 | 27.50 |
| 7841 | 30.00 |
| 8497 | 32.50 |
| 9175 | 35.00 |
| 9881 | 37.50 |
| 10615 | 40.00 |
| 11385 | 42.50 |
| 12189 | 45.00 |
| 13033 | 47.50 |
| 13924 | 50.00 |
| 14870 | 52.50 |
| 15874 | 55.00 |
| 16947 | 57.50 |
| 18096 | 60.00 |
| 19333 | 62.50 |
| 20685 | 65.00 |
| 22167 | 67.50 |
| 23793 | 70.00 |
| 25616 | 72.50 |
| 27661 | 75.00 |
| 29973 | 77.50 |
| 32624 | 80.00 |
| 35745 | 82.50 |
| 39445 | 85.00 |
| 44003 | 87.50 |
| 49720 | 90.00 |
| 57401 | 92.50 |
| 68831 | 95.00 |
| 90432 | 97.50 |

Table 6

| Molecular Weight Distribution | |
|---|---|
| MOLECULAR WEIGHTS | INTEGRAL DISTRIBUTION |
| 146 | 0.00 |
| 156 | 2.50 |
| 175 | 5.00 |
| 197 | 7.50 |
| 223 | 10.00 |
| 250 | 12.50 |
| 279 | 15.00 |
| 311 | 17.50 |
| 345 | 20.00 |
| 381 | 22.50 |
| 420 | 25.00 |
| 462 | 27.50 |
| 506 | 30.00 |
| 555 | 32.50 |
| 603 | 35.00 |
| 662 | 37.50 |
| 721 | 40.00 |
| 792 | 42.50 |
| 875 | 45.00 |
| 971 | 47.50 |
| 1099 | 50.00 |
| 1269 | 52.50 |
| 1496 | 55.00 |
| 1827 | 57.50 |
| 2320 | 60.00 |
| 3043 | 62.50 |
| 4107 | 65.00 |
| 5556 | 67.50 |
| 7396 | 70.00 |
| 9581 | 75.00 |
| 12065 | 75.00 |
| 14880 | 77.50 |
| 18153 | 80.00 |
| 21986 | 82.50 |
| 26590 | 85.00 |
| 32293 | 87.50 |
| 39532 | 90.00 |
| 49285 | 92.50 |
| 63509 | 95.00 |
| 89961 | 97.50 |
| 439968 | 100.00 |

Table 7

| Molecular Weight Distribution | |
| --- | --- |
| MOLECULAR WEIGHTS | INTEGRAL DISTRIBUTION |
| 1586 | 2.50 |
| 2290 | 5.00 |
| 2882 | 7.50 |
| 3443 | 10.00 |
| 3991 | 12.50 |
| 4545 | 15.00 |
| 5110 | 17.50 |
| 5694 | 20.00 |
| 6302 | 22.50 |
| 6931 | 25.00 |
| 7587 | 27.50 |
| 8263 | 30.00 |
| 8965 | 32.50 |
| 9692 | 35.00 |
| 10441 | 37.50 |
| 11218 | 40.00 |
| 12030 | 42.50 |
| 12878 | 45.00 |
| 13761 | 47.50 |
| 14691 | 50.00 |
| 15671 | 52.50 |
| 16705 | 55.00 |
| 17805 | 57.50 |
| 18982 | 60.00 |
| 20244 | 62.50 |
| 21615 | 65.00 |
| 23120 | 67.50 |
| 24766 | 70.00 |
| 26584 | 72.50 |
| 28624 | 75.00 |
| 30930 | 77.50 |
| 33568 | 80.00 |
| 36623 | 82.50 |
| 40240 | 85.00 |
| 44626 | 87.50 |
| 50148 | 90.00 |
| 57346 | 92.50 |
| 67788 | 95.00 |
| 86399 | 97.50 |

Table 8

| Starch Hydrolysate Molecular Weight Distribution | |
| --- | --- |
| MOLECULAR WEIGHTS | INTEGRAL DISTRIBUTION |
| 146 | 0.00 |
| 160 | 2.50 |
| 176 | 5.00 |
| 195 | 7.50 |
| 217 | 10.00 |
| 240 | 12.50 |
| 264 | 15.00 |
| 291 | 17.50 |
| 322 | 20.00 |
| 354 | 22.50 |
| 390 | 25.00 |
| 428 | 27.50 |
| 470 | 30.00 |
| 511 | 32.50 |
| 558 | 35.00 |
| 605 | 37.50 |
| 657 | 40.00 |
| 714 | 42.50 |
| 772 | 45.00 |
| 852 | 47.50 |
| 934 | 50.00 |
| 1050 | 52.50 |
| 1185 | 55.00 |
| 1398 | 57.50 |
| 1688 | 60.00 |
| 2104 | 62.50 |
| 2708 | 65.00 |
| 3617 | 67.50 |
| 4870 | 70.00 |
| 6517 | 75.00 |
| 8552 | 75.00 |
| 10946 | 77.50 |
| 13729 | 80.00 |
| 17036 | 82.50 |
| 21022 | 85.00 |
| 25964 | 87.50 |
| 32324 | 90.00 |
| 40911 | 92.50 |
| 53516 | 95.00 |
| 76329 | 97.50 |
| 356145 | 100.00 |

Table 9

| Time | Pressure | | Temp °C | Permeate Flow Rate l/min | Feed Soln Concn % w/v | Permeate Conc % w/v |
|---|---|---|---|---|---|---|
| | in | out | | | | |
| | Bar | | | | | |
| 0.75 | 4.7 | 3.3 | 67 | 225 | 9.5 | 5.0 |
| 1.25 | 4.7 | 3.8 | 68 | 184 | 10.5 | 5.5 |
| 2.50 | 4.8 | 3.2 | 70 | 150 | 9.0 | 4.0 |
| 3.50 | 4.8 | 3.2 | 70 | 144 | 8.0 | 1.5 |
| 4.50 | 4.8 | 3.2 | 69 | 130 | 6.5 | 0.5 |
| 5.50 | 4.8 | 3.2 | 69 | 123 | 6.0 | 0 |

Table 10

| Permeate (Ex 5a) Molecular Weight Distribution | |
|---|---|
| MOLECULAR WEIGHTS | INTEGRAL DISTRIBUTION |
| 146 | 0.00 |
| 170 | 2.50 |
| 207 | 5.00 |
| 251 | 7.50 |
| 293 | 10.00 |
| 335 | 12.50 |
| 378 | 15.00 |
| 423 | 17.50 |
| 469 | 20.00 |
| 516 | 22.50 |
| 566 | 25.00 |
| 616 | 27.50 |
| 660 | 30.00 |
| 720 | 32.50 |
| 773 | 35.00 |
| 827 | 37.50 |
| 882 | 40.00 |
| 939 | 42.50 |
| 1004 | 45.00 |
| 1070 | 47.50 |
| 1135 | 50.00 |
| 1226 | 52.50 |
| 1320 | 55.00 |
| 1418 | 57.50 |
| 1567 | 60.00 |
| 1717 | 62.50 |
| 1947 | 65.00 |
| 2218 | 67.50 |
| 2566 | 70.00 |
| 3056 | 72.50 |
| 3718 | 75.00 |
| 4671 | 77.50 |
| 5959 | 80.00 |
| 7656 | 82.50 |
| 9753 | 85.00 |
| 12271 | 87.50 |
| 15332 | 90.00 |
| 19237 | 92.50 |
| 24688 | 95.00 |
| 34400 | 97.50 |
| 98105 | 100.00 |

Table 11

| Molecular Weight Distribution | |
| --- | --- |
| MOLECULAR WEIGHTS | INTEGRAL DISTRIBUTION |
| 170 | 0.00 |
| 845 | 2.50 |
| 1292 | 5.00 |
| 1674 | 7.50 |
| 2044 | 10.00 |
| 2429 | 12.50 |
| 2841 | 15.00 |
| 3283 | 17.50 |
| 3754 | 20.00 |
| 4269 | 22.50 |
| 4805 | 25.00 |
| 5361 | 27.50 |
| 5958 | 30.00 |
| 6583 | 32.50 |
| 7232 | 35.00 |
| 7937 | 37.50 |
| 8666 | 40.00 |
| 9447 | 42.50 |
| 10273 | 45.00 |
| 11129 | 47.50 |
| 12062 | 50.00 |
| 13024 | 52.50 |
| 14053 | 55.00 |
| 15147 | 57.50 |
| 16281 | 60.00 |
| 17537 | 62.50 |
| 18860 | 65.00 |
| 20264 | 67.50 |
| 21839 | 70.00 |
| 23542 | 72.50 |
| 25408 | 75.00 |
| 27488 | 77.50 |
| 29900 | 80.00 |
| 32694 | 82.50 |
| 36020 | 85.00 |
| 40183 | 87.50 |
| 45419 | 90.00 |
| 52731 | 92.50 |
| 64063 | 95.00 |
| 85249 | 97.50 |
| 349210 | 100.00 |

## Claims
### Claims for the following Contracting States : BE, CH, DE, FR, GB, IT, LI, LU, NL, SE

1. A peritoneal dialysis composition containing an osmotic agent which is a mixture of glucose polymers, wherein at least 50% by weight of the mixture comprises polymers having molecular weights in the range of from 5,000 to 30,000, and wherein the mixture has a weight average molecular weight of from 5,000 to 50,000 and a number average molecular weight of from 2,900 to 8,000, both the weight average molecular weight and the number average molecular weight being determined using chromatographic columns calibrated with dextran standards (Alsop et al, Process Biochem [2], 10-15 (1977) and Alsop et al, J. Chromatography, [246], 227-240 (1982)).

2. A composition according to claim 1, wherein the mixture of glucose polymers contains less than 5% by weight of polymers of molecular weight greater than 100,000.

3. A composition according to claim 1 or claim 2, wherein the mixture of glucose polymers contains no more than 20% by weight of polymers having molecular weights of from 800 to 10,000.

4. A composition according to any one of the preceding claims, additionally comprising a pharmaceutically acceptable adjuvant, diluent or carrier.

**Claims for the following Contracting State : AT**

1. A process for the preparation of a physiologically acceptable polysaccharide, being a mixture of glucose polymers, wherein at least 50% by weight of the mixture comprises polymers having molecular weights in the range of from 5,000 to 30,000, and wherein the mixture has a weight average molecular weight of from 5,000 to 50,000 and a number average molecular weight of from 2,900 to 8,000, both the weight average molecular weight and the number average molecular weight being determined using chromatographic columns calibrated with dextran standards (Alsop et al, Process Biochem [2], 10-15 (1977) and Alsop et al, J. Chromatography, [246], 227-240 (1982)), characterised in that the process comprises:
   (a) fractional precipitation of an aqueous solution of a polymer with a water miscible solvent, and/or
   (b) filtration of an aqueous solution of a polymer through membranes possessing an appropriate molecular weight cut-off range.

2. A process according to Claim 1, wherein the polysaccharide has a content of glucose polymers with molecular weight greater than 100,000 of less than 5% by weight.

3. A process according to Claim 1 or Claim 2, wherein the polysaccharide has up to 20% by weight of its molecules of a molecular weight of from 800 to 10,000.

4. A process according to any one of the preceding claims, wherein the polysaccharide is admixed with a pharmaceutically acceptable adjuvant, diluent or carrier to provide a composition which is capable of dialysing normal human serum.

**Patentansprüche**
**Patentansprüche für folgende Vertragsstaaten : BE, CH, DE, FR, GB, IT, LI, LU, NL, SE**

1. Eine Peritonealdialyse-Zusammensetzung, die ein osmotisches Mittel enthält, welches eine Mischung von Glukosepolymeren ist, wobei mindestens 50 Gewichts-% der Mischung Polymere mit Molekulargewichten im Bereich von 5000 bis 30000 enthält und wobei die Mischung ein Durchschnittsgewicht des Molekulargewichts von 5000 bis 50000 und eine Durchschnittszahl des Molekulargewichts von 2900 bis 8000 besitzt, wobei sowohl das Durchschnittsgewicht des Molekulargewichts und die Durchschnittszahl des Molekulargewichts mit chromatographischen Säulen bestimmt wird, die mit Dextran Standards geeicht worden sind (Alsop et al, Process Biochem. [2], 10-15 (1977) und Alsop et al., J. Chromatography, [246], 227-240 (1982)).

2. Eine Zusammensetzung nach Anspruch 1, in der die Mischung der Glukosepolymere weniger als 5 Gewichts-% an Polymeren mit einem Molekulargewicht größer als 100000 enthält.

3. Eine Zusammensetzung nach Anspruch 1 oder Anspruch 2, in der die Mischung der Glukosepolymere nicht mehr als 20 Gewichts-% an Polymeren mit Molekulargewichten von 800 bis 10000 enthält.

4. Eine Zusammensetzung nach einem der vorhergehenden Ansprüchen, die zusätzlich ein pharmazeutisch annehmbares Adjuvans, ein Verdünnungsmittel oder einen Träger enthält.

**EP 0 207 676 B1**

**Patentansprüche für folgenden Vertragsstaat : AT**

1. Ein Verfahren für die Herstellung eines physiologisch annehmbaren Polysaccharids, der eine Mischung von Glukosepolymeren ist, wobei mindestens 50 Gewichts-% der Mischung Polymere enthält, die Molekulargewichte im Bereich von 5000 bis 30000 besitzen, und wobei die Mischung ein Durchschnittsgewicht des Molekulargewichts von 5000 bis 50000 besitzt und eine Durchschnittszahl des Molekulargewichts von 2900 bis 8000, wobei sowohl das Durchschnittsgewicht des Molekulargewichts als auch die Durchschnittszahl des Molekulargewichts mit Hilfe von chromatographischen Säulen bestimmt wird, die mit Dextran Standards geeicht worden sind (Alsop et al., Process Biochem [2], 10-15 (1977) und Alsop et al., J. Chromatography [246], 227-240 (1982)), charakterisiert dadurch, daß das Verfahren beinhaltet:
   (a) die fraktionelle Fällung einer wäßrigen Lösung eines Polymers mit einem wassermischbaren Lösungsmittel, und/oder
   (b) die Filtration einer wäßrigen Lösung eines Polymers durch Membranen, die einen geeigneten Molekulargewicht-Rückhaltevermögensbereich besitzen.

2. Ein Verfahren nach Anspruch 1, in dem der Polysaccharid einen Inhalt von weniger als 5 Gewichts-% an Glukosepolymeren mit einem Molekulargewicht größer als 100000 besitzt.

3. Ein Verfahren nach Anspruch 1 oder Anspruch 2, in dem der Polysaccharid bis zu 20 Gewichts-% seiner Moleküle mit einem Molekulargewicht von 800 bis 10000 besitzt.

4. Ein Verfahren nach einem der vorhergehenden Ansprüche, in dem der Polysaccharid mit einem pharmazeutisch annehmbaren Adjuvans, einem Verdünnungsmittel oder einem Träger vermischt ist, um eine Zusammensetzung zur Verfügung zu stellen, die fähig ist, normales menschliches Serum zu dialysieren.

**Revendications**
**Revendications pour les Etats contractants suivants : BE, CH, DE, FR, GB, IT, LI, LU, NL, SE**

1. Une composition pour dialyse péritonéale contenant un agent osmotique qui est un mélange de polymères de glucose, dans laquelle au moins 50% de la masse du mélange comprend des polymères ayant des masses moléculaires dans la plage comprise entre 5.000 et 30.000, et dans laquelle le mélange a une masse moléculaire moyenne entre 5.000 et 50.000 et un nombre de masse moléculaire moyenne entre 2.900 et 8.000, la masse moléculaire moyenne et le nombre de masse moléculaire moyenne étant tous deux déterminés à l'aide de colonnes de chromatographie étalonnées avec des standards de dextranes (Alsop et al, Process Biochem (2), 10-15 (1977) et Alsop et al, J. Chromatography, (246), 227-240 (1982)).

2. Une composition selon la revendication 1, dans laquelle le mélange de polymères de glucose contient moins de 5% en masse de polymères de masse moléculaire supérieure à 100.000.

3. Une composition selon l'une des revendications 1 et 2, dans laquelle le mélange de polymères de glucose ne contient pas plus de 20% en masse de polymères ayant des masses moléculaires entre 800 et 10.000.

4. Une composition selon l'une quelconque des revendications précédentes, comprenant en outre un adjuvant, diluant ou porteur pharmaceutiquement compatible.

**Revendications pour l'Etat contractant suivant : AT**

1. Un procédé pour la préparation d'un polysaccharide physiologiquement compatible, étant un mélange de polymères de glucose, dans lequel au moins 50% de la masse du mélange comprend des polymères ayant des masses moléculaires dans la plage comprise entre 5.000 et 30.000, et dans lequel le mélange a une masse moléculaire moyenne comprise entre 5.000 et 50.000 et un nombre de masse moléculaire moyenne entre 2.900 et 8.000, la masse moléculaire moyenne et le nombre de masse moléculaire moyenne étant tous deux déterminés à l'aide de colonnes de chromatographie étalonnées avec des standards de dextranes (Alsop et al, Process Biochem (2), 10-15 (1977) et Alsop

20

et al, J. Chromatography, (246), 227-240 (1982)), caractérisé en ce que le procédé comporte:

(a) une précipitation fractionnée d'une solution aqueuse d'un polymère avec un solvant miscible à l'eau, et/ou

(b) une filtration d'un solution aqueuse d'un polymère à travers des membranes possédant une plage d'arrêt de masses moléculaires appropriée.

2. Un procédé selon la revendication 1, dans lequel le polysaccharide a une quantité de polymères de glucose avec une masse moléculaire supérieure à 100.000, inférieure à 5% en masse.

3. Un procédé selon l'une des revendications 1 et 2, dans lequel le polysaccharide a jusqu'à 20% en masse de ses molécules d'une masse moléculaire entre 800 et 10.000.

4. Un procédé selon l'une quelconque des revendications précédentes, dans lequel le polysaccharide est mélangé avec un adjuvant, diluant ou porteur pharmaceutiquement compatible pour obtenir une composition apte à dialyser un sérum humain normal.

# Fig.1.

CRUDE DEXTRIN SOLUTION
37 1 AT 116° OR
= 23 KG DEXTRIN ← 33 1 85% ALCOHOL

40 1 ─── 85% ALCOHOL →

SUPER I
65 1 AT 55° OR

SYRUP I
5 1 SYRUP AT 92° OR

SUPER II
84 1 AT 13.5° OR

SYRUP II
21 1 AT 50.25° OR ── 75 1 85% ALCOHOL
── 25 1 PYROGEN FREE WATER

SUPER III
103 1 AT 3.5° OR

SYRUP III
13 1 AT 104° OR ── 54 1 85% ALCOHOL
── 14 1 PYROGEN FREE WATER

SUPER IV
69 1 AT 3.4° OR

48 1 85% ALCOHOL →
12 1 PYROGEN FREE WATER →

SYRUP IV
12 1 AT 98° OR

SUPER V
61 1 AT 2° OR

SYRUP V
10.5 1 AT 102.4° OR
= 5.5 KG OF DEXTRIN

# Fig. 2.

```
                    ┌──────────────────────────────┐ ◄─── 695 1 85%
                    │  CRUDE DEXTRIN SOLUTION       │      ALCOHOL
                    │ 590 1 CONTAINING 380.5 KG     │
                    │        DEXTRIN               │ ◄─── 160 1
                    └──────────────────────────────┘      PYROGEN
                                   │                       FREE WATER
                                   ▼
        ┌──────────────────┐                    ┌──────────────────┐
        │    SUPER I        │ ◄── 870 1 85% ALCOHOL │   SYRUP I         │
        │ 1460 1 AT 40.5° OR│                    │  73 KG SYRUP      │
        └──────────────────┘                    └──────────────────┘

        ┌──────────────────┐            ┌──────────────────────┐ ◄─── 300 1 PYROGEN
        │    SUPER II       │            │    SYRUP II          │      FREE WATER
        │ 233 1 AT 12.5° OR │            │ 291 1 AT 110.0° OR   │ ◄─── 1200 1 85%
        └──────────────────┘            └──────────────────────┘      ALCOHOL

        ┌──────────────────┐            ┌──────────────────────┐ ◄─── 225 1 PYROGEN
        │    SUPER III      │            │    SYRUP III         │      FREE WATER
        │ 1596 1 AT 5.8° OR │            │ 225 1 AT 82.88° OR   │ ◄─── 450 1 85%
        └──────────────────┘            └──────────────────────┘      ALCOHOL

        ┌──────────────────┐            ┌──────────────────────┐ ◄─── 178 1 PYROGEN
        │    SUPER IV       │            │    SYRUP IV          │      FREE WATER
        │ 1272 1 AT 2.48° OR│            │ 178 1 AT 87.6° OR    │ ◄─── 350 1 85%
        └──────────────────┘            └──────────────────────┘      ALCOHOL

        ┌──────────────────┐            ┌──────────────────────┐
        │    SUPER V        │            │    SYRUP V           │
        │                   │            │ 176 KG ≡ 77.4 KG DEXTRIN │
        └──────────────────┘            └──────────────────────┘
```

## Fig. 3.

ETHANOL FRACTIONATION

# Fig.3.(cont).

# Fig.4.

```
600 KG MALTODEXTRIN     ◄─── PYROGEN FREE WATER
TOTAL VOLUME 1500L      ◄─── 3750L 85% ETHANOL
```

```
SUPER I              SYRUP I          ◄── 2554L PYROGEN
4550L AT 8.4° OR     1100L AT 74° OR       FREE WATER
                                      ◄── 2610L 85%
                                          ETHANOL
```

```
SUPER II             SYRUP II             400L PYROGEN
2778L AT 2.6° OR     1135L AT 54.6°OR          FREE WATER
                                          400L 85%
                                          ETHANOL
```

```
SUPER III        ◄── 2900L 85%      SYRUP III
1658L AT 28.5° OR     ETHANOL
```

```
SUPER IV             SYRUP IV         ◄─── 590L PYROGEN FREE WATER
4258L AT 2.4° OR     300L AT 38° OR
```

# Fig.4.(cont).

CARBON TREATMENT
890L , 20 KG ACT.CARBON

←—2200L 85% ETHANOL

SUPER $\overline{V}$
2870L AT 0.9°OR

SYRUP $\overline{V}$
750L AT 57.8° OR

CARBON TREAT,
ION EXCHANGE

SPRAYDRY
163 KG POWDER

27

# Fig.5.

STARCH

STARCH
HYDROLYSATE

HIGH MOLECULAR
WEIGHT FRACTIONATION

ULTRAFILTRATION → RETAINED
PRODUCT

PERMEATE
PRODUCT

LOW MOLECULAR
WEIGHT FRACTIONATION

ULTRAFILTRATION → RETAINED
PRODUCT

PERMEATE
PRODUCT

LOW MOLECULAR
WEIGHT FRACTION